# FASCICULE DE BREVET EUROPEEN

(11) **EP 0 577 464 B1**
(45) Date de publication et mention de la délivrance du brevet: **09.04.2008**
(21) Numéro de dépôt: 93401577.7
(22) Date de dépôt: 21.06.1993
(51) Int. Cl.: C07C 43/04, C07C 41/06, C07C 41/42

(54) **Procédé de préparation d'un éther alkylique tertiaire comprenant une section réactionnelle de finition**
Endarbeitungsreaktionseinheit enthaltendes Verfahren zur Herstellung eines Tertiäralkylethers
Process for the preparation of a tertiary alkyl ether comprising a finishing reaction zone

(30) Priorité: 01.07.1992 FR 9208190
(43) Date de publication de la demande: 05.01.1994
(73) Titulaire: Institut Français du Pétrole, 92502 Rueil-Malmaison (FR)
(72) Inventeur: Nocca, Jean-Luc, Houston, TX 77042 (US); Mank, Larry, F-78630 Orgeval (FR); Travers, Philippe, F-92500 Rueil Malmaison (FR); Forestière, Alain, F-69390 Vernaison (FR)

(56) Documents cités:
- EP-A- 0 082 316
- EP-A- 0 537 636
- WO-A-92/03401
- FR-A- 2 272 063
- FR-A- 2 381 010
- GB-A- 2 047 706
- US-A- 4 988 366

## Description

La présente invention concerne un procédé amélioré pour la fabrication d'un éther alkylique tertiaire par réaction d'au moins une iso-oléfine avec un monoalcool aliphatique en excès. Elle concerne plus particulièrement la fabrication d'éthers alkyliques tertiaires à partir d'au moins un isopentène (ou iso-amylène) et de méthanol, ou d'autres mono-alcools aliphatiques tels que l'éthanol, le propanol ou l'isopropanol, ainsi que la fabrication d'autres éthers alkyliques tertiaires à partir d'isooléfines en C4, C6 et C7 et desdits monoalcools aliphatiques (méthanol, éthanol, propanol ou isopropanol). Elle concerne encore plus particulièrement la fabrication du méthyl tertio-amyl éther (TAME).

Il est connu de préparer des éthers alkyliques tertiaires en faisant réagir une iso-oléfine généralement contenue dans une fraction d'hydrocarbures avec un alcool aliphatique, utilisé en général en excès, en présence d'un catalyseur acide, par exemple l'acide sulfurique, l'acide fluorhydrique, le chlorure d'aluminium ou le fluorure de bore, ou en présence de matières carbonées contenant des groupes - SO₃ H, par exemple des charbons sulfonés, des résines phénol-formaldéhyde sulfonées, des polymères coumarone-indène sulfonés ou, de préférence, des résines de copolymères styrène-divinylbenzène sulfonées.

On sait depuis longtemps que la réaction entre un monoalcool aliphatique, par exemple de 1 à 3 atomes de carbone, et les oléfines tertiaires est équilibrée et qu'il est donc difficile d'obtenir des taux acceptables de conversion des iso-oléfines pour récupérer les éthers alkyliques tertiaires avec une pureté élevée.

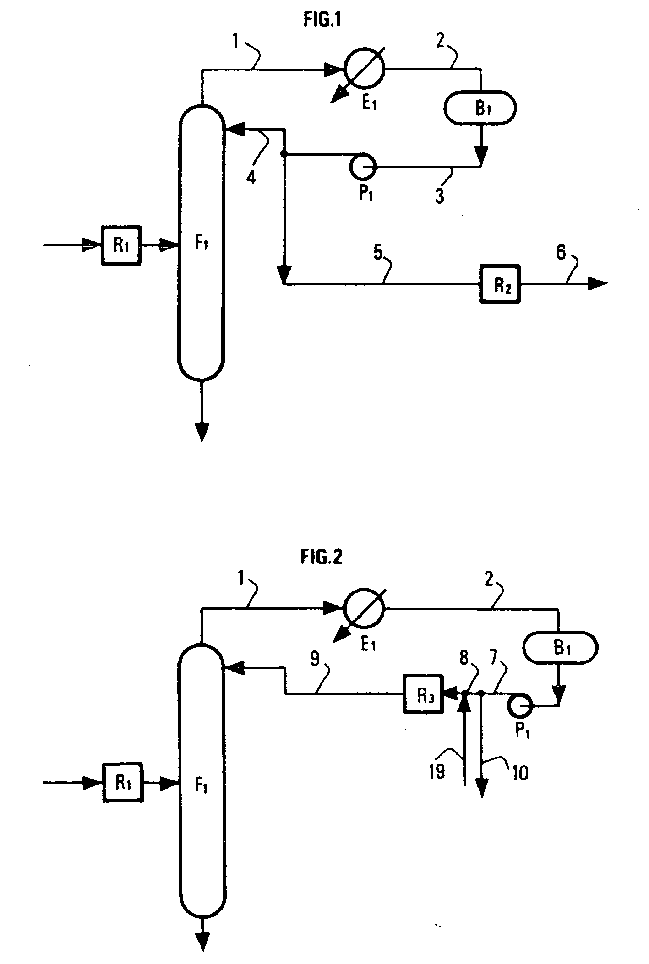

C'est pourquoi, on a déjà préconisé dans l'art antérieur d'ajouter au réacteur principal d'éthérification et à la colonne de fractionnement destinée à recueillir l'éther alkylique tertiaire en fond, une section réactionnelle de finition qui permet d'augmenter dans une certaine mesure, la production de l'éther alkylique tertiaire recherché. Le procédé classique de fabrication d'éther alkylique tertiaire peut être décrit comme suit, en liaison avec la figure 1.

Dans une section réactionnelle principale représentée par le réacteur R₁, on introduit la charge constituée d'un mélange d'hydrocarbures en C4, C5, C6 ou C7 comprenant les isooléfines et d'un monoalcool aliphatique en excès. Les réactifs sont mis contact avec un catalyseur acide.

Le produit issu du réacteur R₁ est envoyé dans une section de fractionnement représentée par la colonne F₁, dans laquelle il est distillé, pour produire en fond un éther alkylique tertiaire pur et en tête un effluent constitué des hydrocarbures non-réactifs contenus dans la charge, des hydrocarbures non-convertis dans le réacteur R₁ et de l'excès de monoalcool aliphatique entrainé par azéotropie ; cet effluent sortant par la ligne 1 étant condensé dans le condenseur E₁ et recueilli par la ligne 2 dans le ballon B₁, d'où il est repris, par la ligne 3, par la pompe P₁, qui envoie une partie de l'effluent vers la tête de la colonne de fractionnement F₁, à titre de reflux par la ligne 4, et le reste de l'effluent, par la ligne 5, vers une section réactionnelle complémentaire, dite de finition, représentée par le réacteur R₂, dans lequel on cherche à faire réagir au contact d'un catalyseur acide la plus forte proportion possible des hydrocarbures non-convertis dans le réacteur principal R₁ avec l'excès de monoalcool aliphatique présent dans l'effluent, de manière à produire une quantité additionnelle d'éther alkylique tertiaire, dans l'effluent sortant du réacteur R₂ par la ligne 6.

Cependant, un tel procédé ne permet pas une conversion très élevée des isooléfines et la proportion d'éther alkylique tertiaire récupéré sous forme de produit pur en fond de colonne F₁, par rapport à la quantité d'éther alkylique tertiaire globale produite est insuffisante. Ainsi, dans le cas particulier de la fabrication du TAME, pour une conversion globale des isopentènes de la charge en TAME de l'ordre de 90 à 92 %, une proportion de l'ordre de 75 % seulement des isopentènes convertis est récupérée en TAME pur.

La demande de brevet européen EP-A-82316 décrit un procédé de préparation de méthyl tertio-butyl éther à partir d'une coupe C4 et de méthanol. Ce procédé comprend la mise en oeuvre d'un réacteur de finition sur le reflux obtenu après la division du condensat recueilli en tête de la colonne de fractionnement, où est renvoyé en reflux l'effluent dudit réacteur de finition. Ce procédé connu est représenté à la figure 2.

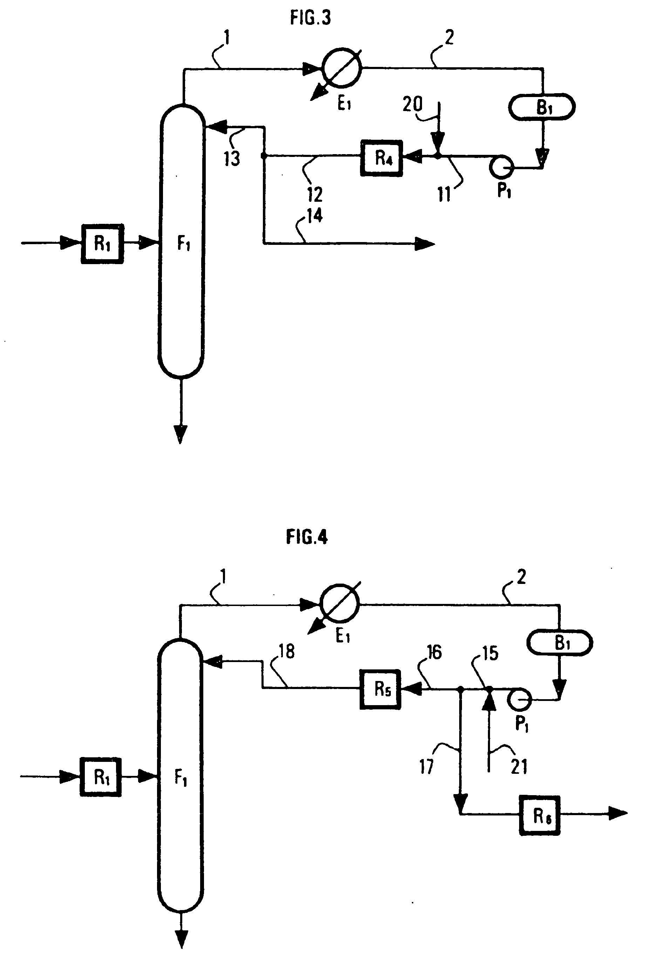

Il serait intéressant de pouvoir augmenter la conversion globale des procédés connus et/ou d'obtenir une récupération plus élevée d'éther alkylique tertiaire pur. Ce sont les objectifs que se fixe l'invention, telle que décrite ci-après, en liaison avec les figures 3 et 4, parmi lesquelles :
- La figure 3 représente schématiquement un premier mode de réalisation du procédé de invention dans lequel le réacteur de finition est à la fois sur le reflux et sur le distillat avant leur division ;et
- La figure 4 représente schématiquement un second mode de réalisation, dans laquelle on dispose un réacteur de finition sur chacun des deux flux : distillat et reflux après leur division.

D'une manière général, le procédé de préparation d'éther alkylique ternaire de l'invention peut être défini par le fait qu'il comprend :
- une première section de réaction dans laquelle on met en contact une charge d'hydrocarbures en C4, C5, C6 ou C7 contenant au moins une isooléfine réactive avec un excès de monoalcool aliphatique, renfermant par exemple de 1 à 3 atomes de carbone ;
- une section de fractionnement dans laquelle on introduit l'effluent de ladite première section de réaction dans une colonne de fractionnement F1, on recueille, en fond, de l'éther alkylique tertiaire pur et, en tête, un effluent qui, après condensation, est divisé en un reflux renvoyé en tête de la colonne de fractionnement et un distillat comprenant des hydrocarbures non-réactifs, des hydrocarbures non-convertis dans la première section de réaction et l'excès de monoalcool aliphatique ;
ledit procédé étant caractérisé en ce qu'il comprend une section réactionnelle complémentaire comprenant
- un réacteur de finition placé sur l'effluent de tête condensé de la colonne de fractionnement, avant sa division;
- ou un premier réacteur de finition sur le reflux de la colonne de fractionnement et un second réacteur de finition sur le distillat de la colonne de fractionnement, après division entre reflux et distillat.

La charge utilisée pour préparer l'éther alkylique tertiaire selon le procédé de l'invention contient en général au moins une isooléfine capable de réagir avec le monoalcool aliphatique dans la réaction d'éthérification donnant naissance à l'éther alkylique tertiaire. Dans le cas de coupe C5, les isooléfines réactives sont plus particulièrement le méthyl-2 butène-1 et le methyl-2 butène 2. En revanche, le méthyl-3 butène-1 (autre isopentène) n'est pas réactif.

Les charges peuvent encore comprendre d'autres hydrocarbures insaturés non-réactifs et des hydrocarbures saturés, de même condensation en carbone que les iso-oléfines réactives.

Le plus souvent, les charges traitées sont des coupes provenant de craquage catalytique et de craquage à la vapeur après fractionnement ; suivant le fractionnement, ces charges pourront contenir de faibles proportions d'hydrocarbures ayant un nombre d'atomes de carbone inférieur ou supérieur à celui des isooléfines à traiter.
Après réaction dans la section réactionnelle R1, menée dans des conditions classiques (phase liquide ou mixte, pression de 2 à 20 bars, de préférence de 5 à 15 bars, température de 40 à 150 °C de préférence de 50 à 100 °C), l'effluent sortant du réacteur R₁ contient en général de l'éther alkylique tertiaire, les hydrocarbures non-réactifs contenus dans la charge, les hydrocarbures non-convertis et l'excès de monoalcool aliphatique. Cet effluent est envoyé dans la section de fractionnement, où il est distillé en général sous une pression absolue de 1 à 10 bars et à une température de fond de 120 à 140 °C.

Dans tous les modes de réalisation de l'invention considérés, l'effluent de tête sortant de la section de fractionnement F1 par la ligne 1 contient les hydrocarbures non-réactifs de la charge, les hydrocarbures non-convertis ainsi que l'excès de monoalcool aliphatique. Cet effluent est condensé dans le condenseur E₁, le condensat, récupéré par la ligne 2 dans le ballon B₁, est repris par la pompe P₁.

Dans un mode de réalisation connu, illustré par la figure 2, le produit sortant de la pompe P1 par la ligne 7 est divisé en deux flux : le reflux et le distillat de la colonne de fractionnement F1.

Le reflux est envoyé par la ligne 8 vers un réacteur de finition R₃, dans lequel les isooléfines non-converties et le monoalcool aliphatique réagissent sur un catalyseur acide pour produire de l'éther alkylique tertiaire.

Comme dans le réacteur R₁, le catalyseur acide utilisé peut être choisi par exemple parmi l'acide sulfurique, l'acide fluorhydrique, le chlorure d'aluminium, le fluorure de bore, des matières carbonées, sulfonées, telles que des charbons sulfonés, des résines phénol-formaldéhyde sulfonées, des polymères coumarone-indène sulfonés ou, de préférence, des résines copolymèes-styrène-divinylbenzène sulfonées. On peut aussi utiliser un catalyseur zéolitique.

La réaction s'effectue en général sur un catalyseur en lit fixe en phase liquide ou mixte une température de 40 à 150 °C, de préférence de 50 à 100 °C, et sous une pression de 2 à 20 bars, de préférence de 5 à 15 bars.

Le produit de réaction est récupéré par la ligne 9 pour être envoyé à titre de reflux en tête de la colonne de fractionnement F₁, l'éther alkylique tertiaire supplémentaire ainsi produit étant récupéré en fond de cette même colonne, conjointement à l'éther alkylique tertiaire produit dans la section réactionnelle principale.

Dans le cas particulier de la fabrication du TAME, la conversion globale des isopentènes obtenue dans ce mode de réalisation est d'environ 85 à 90 %, une proportion de l'ordre de 83 à 85 % des isopentènes convertis étant récupérée en TAME pur.

Le distillat sortant par la ligne 10 contient des hydrocarbures non-réactifs, des hydrocarbures non-convertis, du monoalcool aliphatique non-converti et de faibles quantités d'éther alkylique tertiaire. Il est envoyé en général directement vers une section de lavage et de récupération du monoalcool aliphatique (non représentée sur la figure 2), d'où il sort un raffinat contenant une faible quantité d'éther alkylique tertiaire, correspondant par exemple à 1 à 5 % en poids des isopentènes convertis ; dans le cas particulier de la fabrication du TAME, ce raffinat est utilisable dans le pool essence.

Dans le premier mode de réalisation du procédé de l'invention, illustré par la figure 3, le produit sortant de la pompe P₁ envoyé par la ligne 11 dans un réacteur de finition R₄, dans lequel on opère dans les conditions déjà décrites précédemment. L'effluent sortant du réacteur R₄ par la ligne 12 est divisé en deux flux : le reflux, renvoyé en tête de colonne de fractionnement F₁ par la ligne 13, et le distillat, récupéré par la ligne 14.

Dans le cas particulier de la fabrication de TAME, la conversion globale des isopentènes obtenue est d'environ 94 à 96 %, une proportion de l'ordre de 82 à 85 % des isopentènes convertis étant récupérée en TAME pur.

Le distillat récupéré par la ligne 14 contient les hydrocarbures non-réactifs, les hydrocarbures non-convertis, du monoalcool aliphatique non-réagi et des quantités d'éther alkylique tertiaire qui sont, dans ce cas, plus élevées que celles obtenues dans le mode de réalisation précédent.

Ce distillat est en général envoyé directement vers la section de lavage et de récupération du monoalcool aliphatique, d'où on sort un raffinat contenant, dans le cas particulier de la fabrication du TAME, une proportion de TAME correspondant à 10 à 15 % en poids des isopentènes convertis. Comme précédemment, ce raffinat est utilisable dans le pool essence.

Dans le second mode de réalisation du procédé de l'invention, illustrée par la figure 4, le produit sortant de la pompe P₁ par la ligne 15 est divisé en deux flux : le reflux et le distillat de la colonne.

Le reflux est envoyé par la ligne 16 vers un premier réacteur de finition R₅, le distillat étant envoyé par la ligne 17 vers un second réacteur de finition R₆, chacun des deux réacteurs fonctionnant dans les mêmes conditions que celles décrites précédemment pour le réacteur R₃.

L'effluent issu du réacteur R₅ est envoyé à titre de reflux vers la tête de la colonne de fractionnement F₁ par la ligne 18.

Dans le cas particulier de la fabrication du TAME, la conversion globale des isopentènes en TAME obtenue selon cette variante d'exécution, ainsi que le taux de récupération du TAME pur en fond de colonne de fractionnement F₁, sont du même ordre de grandeur que ceux indiqués pour le second mode de réalisation décrit précédemment.

Pour les deux modes de réalisation du procédé de l'invention, tels que décrits précédemment, on peut envisager d'injecter en amont de chaque réacteur de finition (R₃, R₄ et R₅ + R₆, par les lignes 19, 20 et 21 respectivement) une quantité complémentaire d'au moins un monoalcool aliphatique, qui peut être le même alcool que l'alcool mis en jeu dans le réacteur principal d'éthérification, ou un monoalcool différent, par exemple choisi parmi le méthanol, l'éthanol, le propanol et l'isopropanol. Avantageusement, le second alcool sera plus lourd que celui utilisé dans le réacteur principal d'éthérification R₁. Dans ce cas, on obtiendra, aussi bien en fond de colonne F₁ que dans le raffinat, un mélange d'éthers alkyliques tertiaires, par exemple un mélange de TAME et d'ETAE (éthyl tertioamyl éther), dans lequel le TAME sera en proportion prépondérante.

Par ailleurs, il peut être envisagé de préchauffer la charge de ces réacteurs de finition (R₃, R₄, R₅ et R₆) ainsi que de refroidir les effluents des réacteurs de finition installés sur le reflux (R₃, R₄, et R₅).

Les exemples suivants illustrent l'invention. Les exemples 1 et 2 sont donnés à titre de comparaison.

### EXEMPLES 1 à 4

On a effectué la préparation de TAME, d'une part, selon les schémas classiques (schémas 1 et 2, en liaison avec les figures 1 et 2) et d'autre part selon les modes de réalisation décrits dans l'invention (schémas 3 et 4, en liaison avec les figures 3 et 4 respectivement).

Dans le tableau suivant sont indiqués la composition de la charge et la conversion d'isopentènes dans la section réactionnelle principale R₁ et les conditions opératoires mises en oeuvre dans le(s) réacteur(s) de finition R₃, R₄, R₅ et R₆, et, pour chaque schéma, les conversions obtenues dans le réacteur de finition ainsi que la conversion globale, la quantité de TAME pur récupéré en fond de colonne de fractionnement F₁, et la concentration de TAME dans le raffinat.

**TABLEAU**

| | | **Schéma 1** | **Schéma 2** | **Schéma 3** | **Schéma 4** |
|---|---|---|---|---|---|
| Composition de la charge | | | | | |
| Isopentènes réactifs | | 23 | 23 | 23 | 23 |
| (% poids) | | | | | |
| | | | | | |
| Conversion isopentènes | | 75 | 75 | 75 | 75 |
| Sortie R₁ | | | | | |
| (% poids) | | | | | |
| | | | | | |
| Conditions opératoires | | | | | |
| dans le(s) réacteur(s) de finition : | | | | | |
| | Catalyseur : | résine acide (copolymère styrène-DVB sulfoné) | | | |
| | Type de réacteur: | lit fixe | | | |
| | Phase : | liquide | | | |
| | Pression : | 10 bars | | | |
| | Température : | 65 - 75 °C | | | |
| | | | | | |
| Conversion dans le(s) réacteur(s) de finition | | 68 | 50 | 84 | 50 / 68 |
| (% poids) | | | | | (R₅) (R₆) |
| | | | | | |
| Conversion globale | | 92 | 87,5 | 96 | 96 |
| (% poids) | | | | | |
| | | | | | |
| TAME pur récupéré | | 75 | 84 | 84 | 84 |
| (exprimé en conversion d'isopentènes) | | | | | |
| (% poids) | | | | | |
| | | | | | |
| TAME pur récupéré | | 25,1 | 28,1 | 28,1 | 28,1 |
| (en kg pour 100 kg de charge) | | | | | |
| | | | | | |
| TAME dans raffinat C₅ | | 17 | 3,5 | 12 | 12 |
| (exprimé en conversion d'isopentènes) | | | | | |
| (% poids) | | | | | |
| | | | | | |
| TAME dans raffinat C₅ | | 5,7 | 1,2 | 4 | 4 |
| (en kg pour 100 kg de charge) | | | | | |

On voit que la mise en oeuvre du procédé selon les schémas 3 et 4 permet d'augmenter nettement la proportion de TAME récupéré pur en fond de colonne F1 et d'augmenter également dans une large mesure la conversion globale des isopentènes.

## Revendications

1. Procédé de préparation d'au moins un éther alkylique tertiaire par réaction d'au moins un monoalcool aliphatique et d'au moins une isooléfine, ledit procédé comprenant:
• une première section de réaction dans laquelle on met en contact une charge d'hydrocarbures contenant au moins une isooléfine réactive avec un excès de monoalcool aliphatique,
• une section de fractionnement dans laquelle on introduit l'effluent de ladite première section réactionnelle dans une colonne de fractionnement, on recueille, en fond, de l'éther alkylique tertiaire pur et en tête un effluent qui, après condensation, est divisé en un reflux renvoyé en tête de ladite colonne de fractionnement et en un distillat comprenant des hydrocarbures non réactifs et des hydocarbures non-convertis dans la première section de réaction et l'excès de monoalcool aliphatique,
ledit procédé étant **caractérisé en ce qu'**il comprend une section réactionnelle complémentaire comprenant
• un réacteur de finition placé sur l'effluent de tête condensé de la colonne de fractionnement, avant sa division;
• ou un premier réacteur de finition sur le reflux de la colonne de fractionnement et un second réacteur de finition sur le distillat de la colonne de fractionnement, après division entre reflux et distillat.

2. Procédé selon la revendication 1, **caractérisé en ce que** la charge consiste en une coupe C₄, C₅, C₆ ou C₇.

3. Procédé selon l'une des revendications 1 et 2, **caractérisé en ce que** ledit monoalcool aliphatique est choisi parmi le méthanol, l'éthanol, le propanol et l'isopropanol.

4. Procédé selon l'une des revendications 1 à 3, **caractérisé en ce que** la charge consiste en une coupe C5 et le monoalcool aliphatique est le méthanol, l'éther alkylique tertiaire formé étant le méthyl tertioamyl éther (TAME).

5. Procédé selon l'une des revendications 1 à 4, **caractérisé en ce que**, dans tout réacteur de finition, la réaction est réalisée en phase liquide ou mixte dans un réacteur à lit fixe, à une température de 40 à 150°C et sous une pression de 2 à 20 bars.

6. Procédé selon l'une des revendications 1 à 5, **caractérisé en ce que**, dans tout réacteur de finition, on utilise un catalyseur acide choisi parmi l'acide sulfurique, l'acide fluorhydrique, le chlorure d'aluminium, le fluorure de bore, les matières carbonées sulfonées, les résines phénol-formaldéhyde sulfonées, les polymères coumarone-indène sulfonés et les résines copolymères-styrène-divinylbenzène sulfonées, ou un catalyseur zéolitique.

7. Procédé selon la revendication 6, **caractérisé en ce que** ledit catalyseur acide consiste en une résine copolymère styréne-divinylbenzène sulfonée.

8. Procédé selon l'une des revendications 1 à 7, **caractérisé en ce que** l'on injecte en amont du réacteur de finition un monoalcool aliphatique choisi parmi le méthanol, l'éthanol, le propanol et l'isopropanol.

9. Procédé selon l'une des revendications 1 à 8, **caractérisé en ce que** l'on préchauffe la charge du (ou des) réacteur(s) de finition.

10. Procédé selon l'une des revendications 1 à 9, **caractérisé en ce que** l'on refroidit l'effluent du réacteur de finition installé sur le reflux.

## Claims

1. A process for production of at least one tertiary alkyl ether by reacting at least one aliphatic monoalcohol and at least one isoolefin, said process comprising:
a first reaction section in which a hydrocarbon charge containing at least one reactive isoolefin is contacted with a excess of aliphatic monoalcohol,
• a fractionation zone in which the effluent of said first reaction zone is introduced into a fractionation column, in the bottom pure tertiary alkyl ether is recovered, head effluent is recovered, and after condensation, condensed head effluent is subdivided into a reflux stream which is sent back to the head of said fractionation column and a distillate stream containing non-reactive hydrocarbons, hydrocarbons not converted in said first reaction section and excess aliphatic monoalcohol,
said process being **characterized in that** it comprises a complementary reaction section comprising:
• a finishing reactor positioned on the condensed head effluent of the fractionation column, before the subdivision of said effluent,
• or a first finishing reactor positioned on the reflux stream of the fractionation column and a second finishing reactor positioned on the distillate stream of the fractionation column, after subdivision between reflux and distillate streams.

2. A process according to claim 1, wherein said charge is a C₄, C₅, C₆ or C₇ fraction.

3. A process according to any one of claims 1 or 2, wherein said aliphatic monoalcohol is methanol, ethanol, propanol or isopropanol.

4. A process according to any one of claims 1 to 3, wherein said charge is a C₅ fraction, said aliphatic monoalcohol is methanol, and said produced tertiary alkyl ether is methyl tertioamyl ether (TAME).

5. A process according to any of claims 1 to 4, wherein, any of said finishing reactor is a fixed bed reactor and the reaction conducted in said finishing reactor is performed in the liquid phase or in a mixed phase at a temperature of 40 to 150°C and under a pressure of 2 to 20 bars.

6. A process according to any of claims 1 to 5, wherein any of said finishing reactor contains an acid catalyst selected from the group consisting of sulphuric acid, hydrofluoric acid, aluminum chloride, boron fluoride, sulphonated/carbonated materials, sulphonated phenol-formaldehyde resins, sulphonated coumarone-indene polymers, sulphonated styrene-divinyl benzene copolymer resins and zeolitic catalysts.

7. a process according to claim 6, wherein said acid catalyst is a sulphonated styrene-divinyl benzene copolymer resin.

8. A process according to any of claims 1 to 7, wherein at least one monoaliphatic alcohol selected from the group consisting of methanol, ethanol, propanol and isopropanol is injected upstream of said finishing reactor.

9. A process according to any of claims 1 to 8, wherein the charge of any of said finishing reactor is preheated.

10. A process according to any of claims 1 to 9, wherein the finishing reactor effluent positioned on the reflux stream is cooled.

## Patentansprüche

1. Verfahren zur Herstellung mindestens eines tertiären Alkylesters durch Umsetzung mindestens eines aliphatischen Monoalkohols und mindestens eines Isoolefins, wobei das Verfahren umfasst:
• einen ersten Reaktionsabschnitt, in dem eine Kohlenwasserstoffbeschickung, die mindestens ein reaktives Isoolefin enthält, mit einem Überschuss an aliphatischem Monoalkohol in Kontakt gebracht wird,
• einen Fraktionierungsabschnitt, in dem der Abfluss aus dem ersten Reaktionsabschnitt in eine Fraktionierungssäule eingeführt wird, am Boden tertiärer Alkylether und am Kopf ein Abfluss gesammelt wird, der nach Kondensation, in einen Rückfluss, der zum Kopf der Fraktionierungssäule zurückgeschickt wird, und in ein Destillat geteilt wird, das nicht reaktive Kohlenwasserstoffe und in dem ersten Reaktionsabschnitt nicht umgewandelte Kohlenwasserstoffe und den Überschuss an aliphatischem Monoalkohols umfasst,
wobei das Verfahren **dadurch gekennzeichnet ist, dass** es einen zusätzlichen Reaktionsabschnitt umfasst, der umfasst
• einen Veredelungsreaktor, der auf dem kondensierten Kopfabfluss der Fraktionierungssäule, vor dessen Teilung, angeordnet ist;
• oder einem ersten Veredelungsreaktor auf dem Rückfluss der Fraktionierungskolonne und einem zweiten Veredelungsreaktor auf dem Destillat der Fraktionierungskolonne, nach der Teilung in Rückfluss und Destillat.

2. Verfahren nach Anspruch 1, **dadurch gekennzeichnet, dass** die Beschickung aus einem C₄-, C₅-, C₆- oder C₇-Schnitt besteht.

3. Verfahren nach einem der Ansprüche 1 bis 3, **dadurch gekennzeichnet, dass** der aliphatische Monoalkohol aus Methanol, Ethanol, Propanol und Isopropanol ausgewählt ist.

4. Verfahren nach einem der Ansprüche 1 bis 3, **dadurch gekennzeichnet, dass** die Beschickung aus einem C5-Schnitt besteht und der aliphatische Monoalkohol Methanol ist, wobei der gebildete tertiäre Alkylether tert.-Amylmethylether (TAME) ist.

5. Verfahren nach einem der Ansprüche 1 bis 4, **dadurch gekennzeichnet, dass** in jedem Veredelungsreaktor die Umsetzung in flüssiger oder gemischter Phase in einem Festbettreaktor bei einer Temperatur von 40 bis 150 °C und unter einem Druck von 2 bis 20 Bar ausgeführt wird.

6. Verfahren nach einem der Ansprüche 1 bis 5, **dadurch gekennzeichnet, dass** in jedem Veredelungsreaktor ein saurer Katalysator verwendet wird, der aus Schwefelsäure, Fluorwasserstoffsäure, Aluminiumchlorid, Borfluorid, sulfonierten Kohlenstoffmaterialien, sulfonierten Phenolformaldehydharzen, sulfonierten Cumaron-Inden-Polymeren und sulfonierten Styrol-Divinylbenzol-Copolymerharzen oder einem zeolithischen Katalysator ausgewählt ist.

7. Verfahren nach Anspruch 6, **dadurch gekennzeichnet, dass** der saure Katalysator aus einem sulfonierten Styrol-Divinylbenzol-Copolymerharz besteht.

8. Verfahren nach einem der Ansprüche 1 bis 7, **dadurch gekennzeichnet, dass** stromaufwärts des Veredelungsreaktors ein aliphatischer Monoalkohol eingespritzt wird, der aus Methanol, Ethanol, Propanol und Isopropanol ausgewählt ist.

9. Verfahren nach einem der Ansprüche 1 bis 8, **dadurch gekennzeichnet, dass** die Beschickung des (oder der) Veredelungsreaktors (Veredelungsreaktoren) vorerwärmt wird.

10. Verfahren nach einem der Ansprüche 1 bis 9, **dadurch gekennzeichnet, dass** der Abfluss des Veredelungsreaktors, der auf dem Rückfluss angebracht ist, abgekühlt wird.
